# EUROPEAN PATENT APPLICATION

(11) **EP 4 844 404 A1**
(43) Date of publication of application: **25.11.2026**
(21) Application number: 26179125.5
(22) Date of filing: 18.05.2026
(51) Int. Cl.: G01N 33/49, G01N 21/27, G01N 21/47, G01N 21/51

(54) **SYSTEM AND METHOD FOR CALIBRATION OF AN OPTICAL CELL CONCENTRATION MEASUREMENT SENSOR**

(30) Priority: 19.05.2025 US 202563808390 P
(71) Applicant: Fenwal, Inc., Lake Zurich, IL 60047 (US)
(72) Inventor: KUSTERS, Benjamin, Lake Zurich, 60047 (US)
(74) Representative: Maikowski & Ninnemann Patentanwälte Partnerschaft mbB

(57) **Abstract**

Devices and methods for calibrating an optical sensing system. In particular, devices and methods including placing a scattering material on a fluid vessel, wherein the scattering material is configured to provide a preset reference signal.

## Description

### FIELD OF DISCLOSURE

The present disclosure relates generally to the optical monitoring of fluids. More particularly, the present disclosure relates to systems and methods for calibrating an optical sensor system used in biological fluid processing procedures.

### BACKGROUND

In the field of biological fluid processing, optical detection assemblies may be utilized to monitor the flow of blood, blood components, and/or other biological fluids through a fluid flow circuit to determine various characteristics of the fluid. A typical optical detection assembly includes a light source (e.g., a laser or a light-emitting diode) configured to emit light into a fluid-containing vessel of the fluid flow circuit, with a light detector (e.g., a photodiode) configured to receive light exiting the vessel. The light detector transmits a signal to a coupled controller based upon the light it received, with the controller using the signal to determine one or more properties of the fluid (and, if necessary, adjusting or otherwise initiating certain actions in the biological fluid processing procedure).

When using an optical detection assembly, it is desirable (if not imperative) that the sensor is properly calibrated to provide reliable information. For instance, when optically measuring the concentration of cells flowing through a vessel using scattering properties of the fluid, the sensor must operate within specifications, including proper incident light intensity, vessel alignment, and optical hardware (*i.e.*, light source, sensor, etc.) alignment.

Often, sensor calibration involves expensive equipment. Such cost constraints may motivate an operator to calibrate only at pre-determined scheduled intervals to save on costs. Accordingly, a sensor assembly may not be calibrated at a sufficient frequency and, as a result, may provide imprecise information before its scheduled calibration. When using a sensor assembly to determine concentration of cells in a fluid, imprecise information from an uncalibrated sensor can lead to incorrect determinations of concentration.

Therefore, there exists a need for improved and reliable systems and methods to calibrate an optical detection assembly.

### SUMMARY

There are several aspects of the present subject matter which may be embodied separately or together in the devices, systems, and methods described and/or claimed below. These aspects may be employed alone or in combination with other aspects of the subject matter described herein, and the description of these aspects together is not intended to preclude the use of these aspects separately or the claiming of such aspects separately or in different combinations as set forth in the claims appended hereto or later amended. For purposes of this description and claims, unless otherwise expressly indicated, "blood" is intended to include whole blood and blood components, such as concentrated red cells, plasma, platelets and white cells, whether with or without anticoagulant or additives.

In one aspect, a system for calibrating an optical sensor used in a blood processing procedure is provided. The system includes a fluid vessel including a fluid passage configured to accommodate a biological fluid and an optical sensing system associated with the fluid vessel. The optical sensing system includes a light source and an optical sensor. The system for calibrating an optical sensor also includes a scattering material located on a surface of the fluid vessel.

In another aspect, a method of calibrating an optical sensing system is provided. The method includes attaching a scattering material onto a surface of a fluid vessel. The fluid vessel includes a fluid passage configured to accommodate a biological fluid and the scattering material is configured to produce a preset reference signal when exposed to light including known properties. The method further includes placing the fluid vessel in an optical sensing system. The optical sensing system includes a light source and an optical sensor. Additionally, the method includes passing a light from the light source through the fluid vessel and comparing a collected signal from the optical sensor to the preset reference signal.

In another aspect, a method of processing a blood product is provided. The method includes loading a blood processing circuit onto a blood processing device. The blood processing circuit includes a fluid vessel configured to accommodate a flow of a biological fluid. The method also includes attaching a scattering material onto a surface of the fluid vessel. The scattering material is configured to produce a preset reference signal when exposed to a light including known properties. The method further includes placing the fluid vessel in an optical sensing system. The optical sensing system includes a light source and an optical sensor. Furthermore, the method includes passing a light from the light source through the fluid vessel, comparing a collected signal from the optical sensor with the preset reference signal, adjusting the light source if the collected signal is not similar to the preset reference signal, and executing a blood processing procedure.

These and other aspects of the present subject are set forth in the following detailed description of the accompanying drawings.

### BRIEF DESCRIPTION OF THE DRAWINGS

FIG. 1 is a perspective plan view of an example of an optical sensing system.
FIG. 2 is a perspective view of an example of a fluid vessel.
FIG. 3 is a perspective view of the fluid vessel of FIG. 1 including an example of a scattering material.
FIG. 4 is an exploded perspective view of the fluid vessel of FIG. 1 and an example of a scattering material.
FIG. 5 a perspective view of the fluid vessel of FIG. 1 and the scattering material of FIG. 4 in an attached configuration.
FIG. 6 is a plan view of an example of an optical sensing system during calibration.
FIG. 7 is a graph showing a reference signal.
FIG. 8A depicts a pair of graphs showing a reference signal and a collected calibration signal, respectively, wherein the reference signal and collected calibration signal are similar.
FIG. 8B depicts a pair of graphs showing a reference signal and a collected calibration signal, respectively, wherein the reference signal and collected calibration signal are similar.
FIG. 8C depicts a pair of graphs showing a reference signal and a collected calibration signal, respectively, wherein the reference signal and collected calibration signal are similar.
FIG. 9 depicts a pair of graphs showing a reference signal and a collected calibration signal, respectively, wherein the reference signal and collected calibration signal are dissimilar.
FIG. 10 is a flowchart of a method of calibrating an optical sensing system.

### DETAILED DESCRIPTION OF THE EMBODIMENTS

The embodiments disclosed herein are for the purpose of providing an exemplary description of the present subject matter. They are, however, only exemplary and not exclusive, and the present subject matter may be embodied in various forms. Therefore, specific details disclosed herein are not to be interpreted as limiting the subject matter as defined in the accompanying claims.

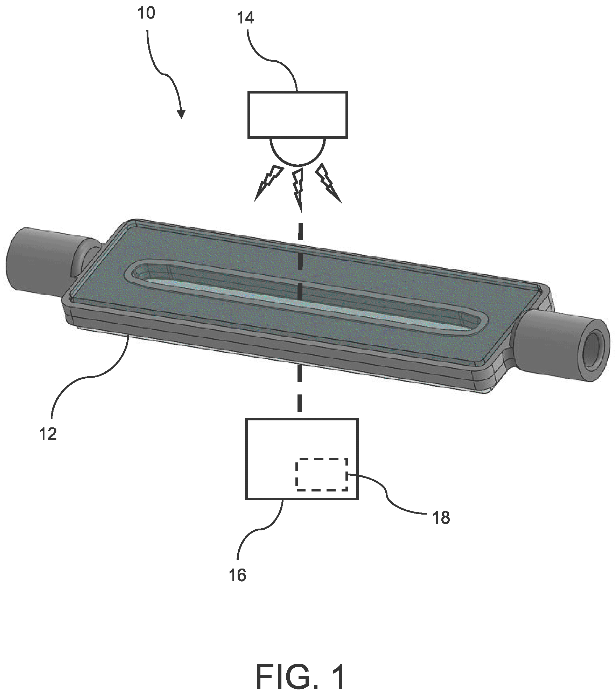

FIG. 1 illustrates an example of an optical sensing system 10 including a fluid vessel 12, a light source 14, and a light detector 16. The fluid vessel 12 can be used with and/or incorporated into a disposable fluid circuit, the disposable fluid circuit configured to be associated with a reusable hardware component of a biological fluid processing system or device. The optical sensing system 10 can be used to measure characteristics of fluid flowing through the disposable fluid circuit during a biological fluid processing procedure, such as a blood processing procedure. For example, the optical sensing system 10 can be used to detect the presence of fluid in a portion of the disposable circuit and to measure characteristics of the fluid such as, but not limited to, flow rate, opacity, hematocrit, volume, and any other suitable characteristics. In a more particular example, the optical sensing system 10 can be configured to measure the concentration of separated cells (e.g., red blood cells (RBCs)) flowing through the disposable circuit. It will be understood that the optical sensing system 10 can be used to detect various characteristics of various biological or other fluids travelling through a fluid circuit without departing from the scope of the disclosure.

As described above, the optical sensing system 10 can include a light source 14 and a light detector 16. In an example, the light source 14 can be a laser or a light emitting diode. The light detector 16 can be a photodiode or an array of photodiodes. In another example, the optical sensing system 10 can include any other suitable light sources and/or light detectors including, but not limited to, those disclosed in U.S. Publication No. 2023/0243746 A1 and U.S. Publication 2025/0147003 A1, which are incorporated by reference herein. The light source 14 is configured to emit light through a visible fluid flow path within the fluid vessel 12 and the light detector 16 is configured to detect the light transmitted through the visible fluid flow path.

The optical sensing system 10 can include a microprocessor-based controller 18 configured to record and analyze the information received from the light detector 16. Controller 18 includes an operating system which executes apparatus operation software stored in a related memory. Light source 14 or the light detector 16 can include the controller 18, or alternatively, the hardware component of the biological fluid processing system can include the controller 18 and is configured to receive information from the light detector 16. In an example, the optical sensing system 10 can include controller 18, and the hardware component of the processing system can include a separate controller coupled to controller 18, such that information can be transmitted between the optical sensing system 10 and the hardware component. In another example, controller 18 can be an external controller that is coupled to the optical sensing system 10. Additionally, controller 18 can be coupled to a computing device (e.g., a computer, laptop, smart device, etc.) and is configured to transmit and receive information to and from the computing device. It will be understood that coupling the controller 18 includes connecting via a wireless or wired connection.

Turning to FIG. 1, an example of a fluid vessel 12 is shown. The fluid vessel 12 is configured to accommodate a flow of a biological fluid therethrough. In particular, the fluid vessel 12 allows for concurrent fluid flow within the fluid vessel 12 and optical measurement of the fluid flowing therethrough. The fluid vessel 12 is configured to be attached to the tubing that defines a flow path of a disposable fluid circuit, such that the fluid vessel 12 is in line with the tubing of the disposable fluid circuit. For instance, fluid vessel 12 can be attached to the tubing of a disposable fluid circuit configured for blood processing procedures, such as, but not limited to, apheresis. In particular, the fluid vessel 12 can be attached to a fluid flow circuit configured to be used with the hardware component of a system of the type described in U.S. Publication 2025/0147003, the contents of which are incorporated by reference herein in its entirety. Accordingly, the fluid vessel 12 can be configured to receive the flow of blood, separated blood component(s), blood product(s), or other fluid (bodily or non-bodily).

In an example, the fluid vessel 12 includes a body 20 with a fluid passage 22. The fluid passage 22 extends between a first end 24 and a second end 25 of the body 20 and is in fluid communication with an inlet 26 located at the first end 24 and an outlet 28 located at the second end 25. In an example, the inlet 26 can be an inlet tubing port located at the first end 24 of the body 20 and the outlet 28 can be an outlet tubing port located at the second end 25 of the body 20. The inlet tubing port and outlet tubing port can be configured to receive tubing associated with the disposable fluid circuit. In an example, the tubing can be releasably attached to the inlet and outlet tubing ports. For instance, tubing can be attached to the inlet and outlet tubing ports via a push fit, a friction fit, or with a luer fitting, among other suitable releasable attachment means. Alternatively, tubing can be irreversibly attached to the inlet and outlet tubing ports. For instance, tubing can be attached to the inlet and outlet tubing ports via welding, adhesion, or any other suitable irreversible attachment or bonding method.

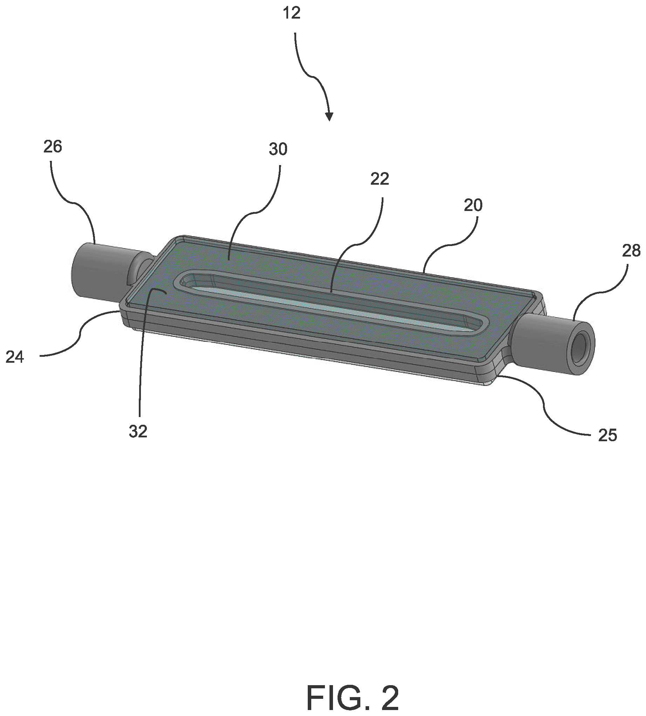

Fluid vessel 12 includes transparent surfaces, allowing for the light detector 16 to detect a light passing through the fluid vessel 12 from the light source 14. As shown in FIG. 2, in an example, the body 20 can have a generally flat, rectangular shape with a first surface 30 and an opposing second surface (not shown). First surface 30 and opposing second surface can include transparent material. For instance, body 20 can include a first transparent wall 32 defining the first surface 30 and a second transparent wall defining the opposing second surface (not shown). The first transparent wall 32 and second transparent wall can be made of a transparent material, such as a transparent polymer or glass. Additionally, the first and second transparent walls can be integral portions of the body 20 or they can be separate pieces that are attached to the body 20. Without departing from the scope of the disclosure, body 20 can be any suitable shape with a transparent surface, allowing fluid flow therethrough and for light to pass through the body 20. In an example, the fluid vessel 12 can be a flow-through cuvette, such as the cuvettes described in Provisional U.S. Application No. 63/736,052 and Provisional U.S. Application No. 63/686,578.

To calibrate the optical sensing system 10, a scattering material 34 can be located on a surface of the fluid vessel 12. The scattering material 34 is configured to provide a preset reference signal when the light source 14 emits a light with known properties therethrough. In other words, when the light source 14 emits a light at particular parameters, such as intensity, wavelength, etc., the scattering material 34 is configured to scatter the light in a predetermined manner. Accordingly, the light detector 16 receives the scattered light and produces a preset reference signal.

In accordance with the present disclosure, the optical sensing system 10 can be calibrated before a biological fluid processing procedure is executed. For instance, the disposable fluid circuit including the fluid vessel 12 is placed on a reusable hardware device including the light source 14 and light detector 16. The fluid vessel 12 is placed between the light source 14 and the light detector 16 and is positioned such that light from the light source 14 is directed through the fluid passage 22.

As described above, the scattering material 34 is applied or otherwise located on a surface of the fluid vessel 12. In an example, the scattering material 34 is made of a semi-transparent material. The scattering material 34 can be located on at least one surface of the fluid vessel 12. Alternatively, the scattering material 34 can be located on all surfaces of the fluid vessel 12.

The scattering material 34 can allow varying amounts of light to pass therethrough. For example, the scattering material may have a transparency of 20-90%. The amount of light passing through the scattering material 34 and fluid passage 22 may depend, in part, on the fluid to be measured within the fluid passage 22 and the intensity of the light from the light source 14. For example, if (pure) plasma is to be measured within the fluid passage 22, the light source may emit a low-intensity light *(e.g.,* a light at an intensity suitable to pass through the scattering material, fluid passage 22, and pure plasma). In this instance, the scattering material 34 may allow up to 90% of the light to pass therethrough (*i.e.,* the scattering material 34 has a transparency of up to about 90%). In a particular example, the scattering material 34 has a transparency of 90%. Alternatively, if platelet concentrate is to be measured within the fluid passage 22, the light source may emit a high-intensity light (e.g., a light at an intensity suitable to pass through the scattering material 34, fluid passage 22, and platelet concentrate). In this instance, the scattering material may allow for about 50% of the light to pass therethrough *(i.e.,* the scattering material 34 has a transparency of less than 90%, more particularly the scattering material 34 has a transparency of about 50%). In a particular example, the scattering material 34 has a transparency of 50%.

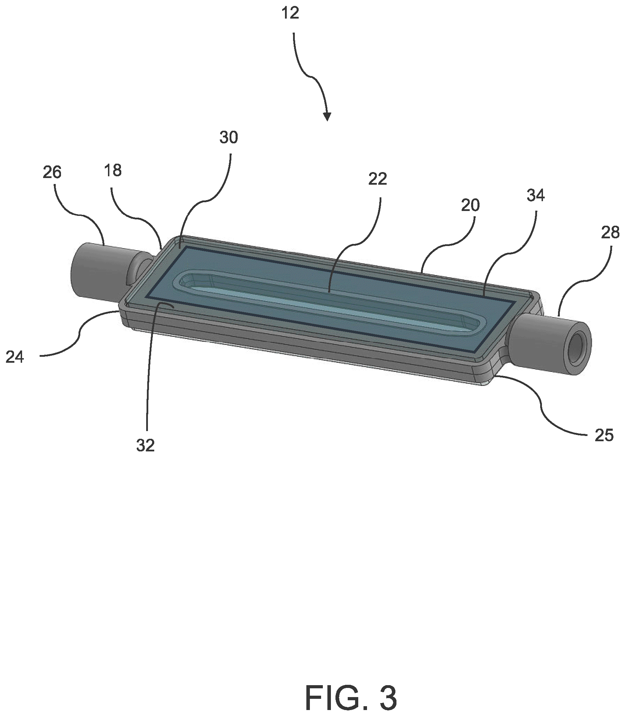

Turning to FIG. 3, fluid vessel 12 includes the scattering material 34. In an example, the scattering material 34 can be a sheet of plastic film. As shown in FIG. 3, the scattering material 34 can be attached or applied to at least one surface of the fluid vessel. For instance, the plastic film can be temporarily adhered to a surface of the fluid vessel. Other temporary attachment mechanisms can be used to attach the scattering material 34 to a surface of the fluid vessel 12 without departing from the scope of the disclosure. In some examples, the film can be attached to the first surface 30 and the second surface (not shown).

As shown in FIG. 3, the film can be sized to cover at least the fluid passage 22. In an example, the film can be sized to cover an entire surface of the fluid vessel 12. In another example, the scattering material 34 can be a film that is sized to cover both the first surface and the second surface. For instance, the film can be attached to the first surface 30 and folded over the fluid vessel 12 and attached to the second surface. In another example, the scattering material 34 can be a flexible sleeve formed of a film. For instance, the sleeve can be slid over the fluid vessel 12 and attached to the fluid vessel 12 before tubing is connected to the inlet 26 and the outlet 28. After the optical sensing system 10 has been calibrated, the sleeve can be removed from the fluid vessel 12. For example, the sleeve can include a perforation configured to tear the sleeve, assisting in removal of the sleeve.

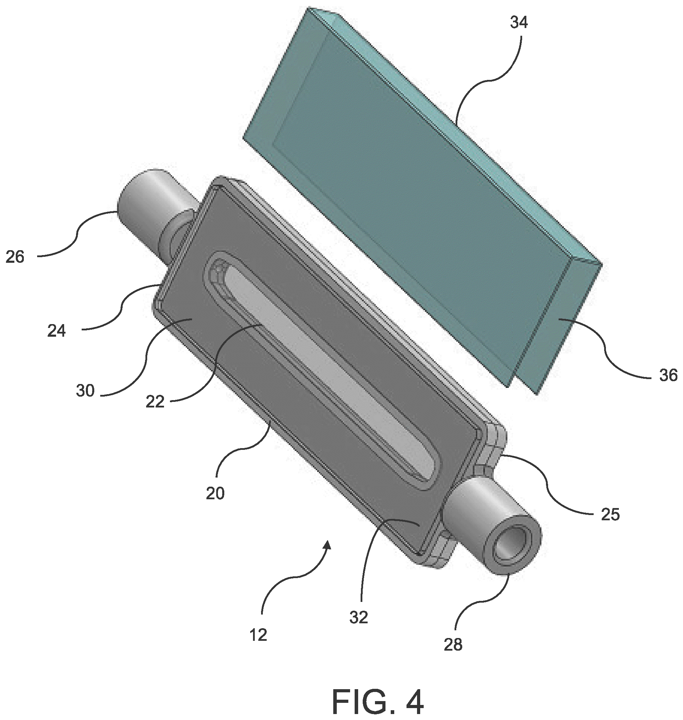

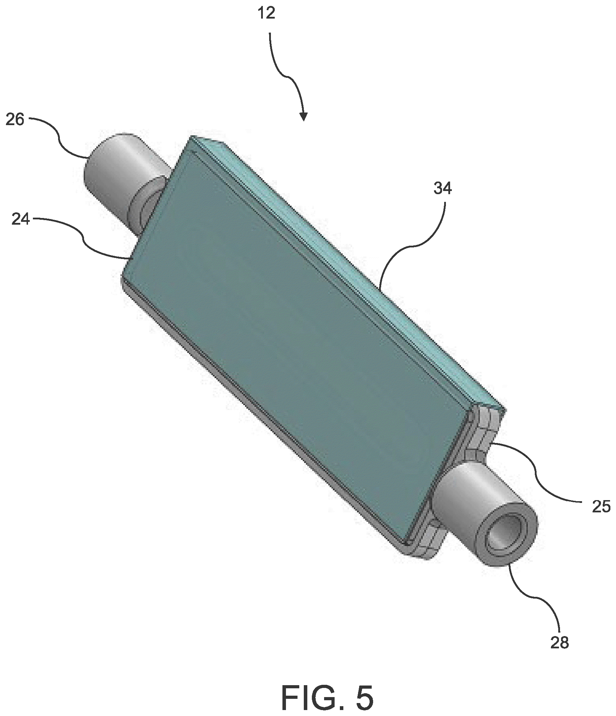

FIGS. 4 and 5 illustrate another example of the scattering material 34. As shown in FIGS. 4 and 5, scattering material 34 can be a sleeve made of rigid material configured to fit over and/or around the fluid vessel 12. For example, the scattering material 34 can be a rigid U-shaped member including a slot 36 to receive the fluid vessel 12. As shown in FIG. 5, the scattering material 34 can be attached to the fluid vessel 12 by sliding it over the fluid vessel 12. The scattering material 34 can be kept in place via a friction-fit between the scattering material 34 and the fluid vessel 12. Alternatively, the scattering material 34 can be attached to the fluid vessel 12 via clamps, fasteners, a snap-fit, or any other suitable attachment mechanism without departing from the scope of the disclosure.

The scattering material 34 can be attached to the fluid vessel 12 before or after attachment of the fluid vessel 12 to a disposable fluid circuit. In an example, the scattering material 34 can be attached to the fluid vessel 12 after manufacturing of the fluid vessel 12 and can prevent damage or dirtying of the transparent surface(s) of the fluid vessel 12. For instance, the scattering material 34 can prevent dust, fingerprints, smudges, or other debris, from dirtying the fluid vessel 12.

After attachment of the fluid vessel 12 to a disposable fluid circuit, the fluid vessel 12 with the scattering material 34 attached thereto is placed onto a reusable hardware device configured to execute the biological fluid processing procedure. As described herein, the fluid vessel 12 is positioned between the light source 14 and light detector 16 of the reusable hardware device and is positioned such that light from the light source 14 is passed through the fluid passage 22.

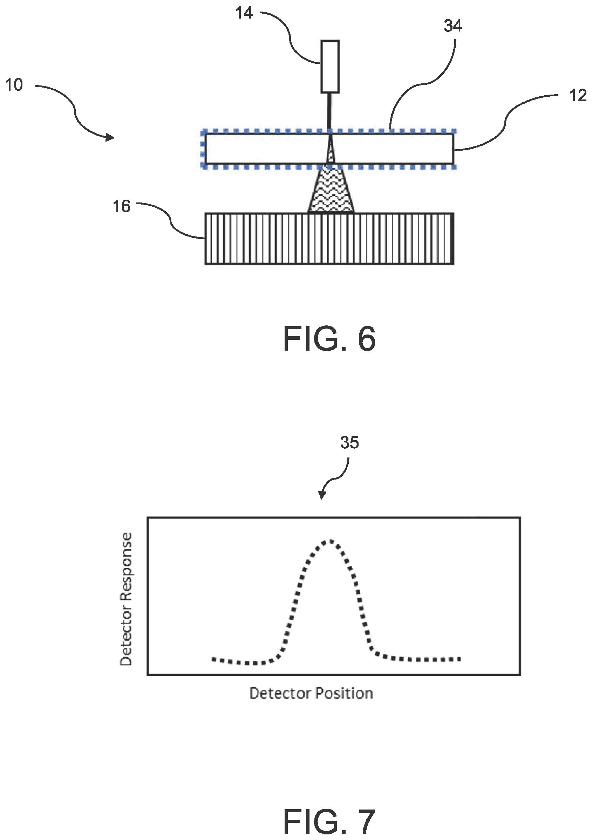

Before commencing the biological fluid processing procedure, the optical sensing system 10 can be calibrated. As shown in FIG. 6, light from the light source 14 is passed through the scattering material 34 and fluid vessel 12. During calibration, the fluid passage 22 is empty. The light detector 16 is configured to detect the light passing through the scattering material 34 and the fluid vessel 12. In an example, as shown in FIG. 6, the scattering material 34 is a U-shaped member as previously described and covers both surfaces of the fluid vessel 12. Thus, the light may pass through the scattering material 34 twice *(i.e.,* the light travels first through the scattering material 34, then through the fluid vessel 12, and then again through the scattering material 34). In another example, the scattering material 34 may only be located on one surface of the fluid vessel 12 (either the first surface 30 or the second surface) and the light passes through the scattering material 34 only once.

The scattering material 34 is configured to produce a preset reference signal when a light with known properties is passed therethrough. FIG. 7A illustrates an example of a reference signal 35 as a function of the light detector position and the light detector response when the light detector 16 is a photodiode array (as shown in FIG. 6). The light detector response can be the raw response output from a detector measured in voltage, the raw response voltage measurement processed by an analog-to-digital (A/D) converter into a digital signal, light intensity calculated from a voltage measurement or digital signal, or any other suitable output or metric received from a light detector. It will be understood that the reference signal can vary and can be plotted as a function of other suitable measurements. The reference signal can be preloaded and saved to controller 18 *(e.g.,* to a memory of controller 18).

During calibration of the optical sensing system 10, a light from light source 14 is passed through the scattering material 34 and fluid vessel 12. The light detector 16 receives the light passing through the scattering material 34 and fluid vessel 12 and sends the collected signal to the controller 18.

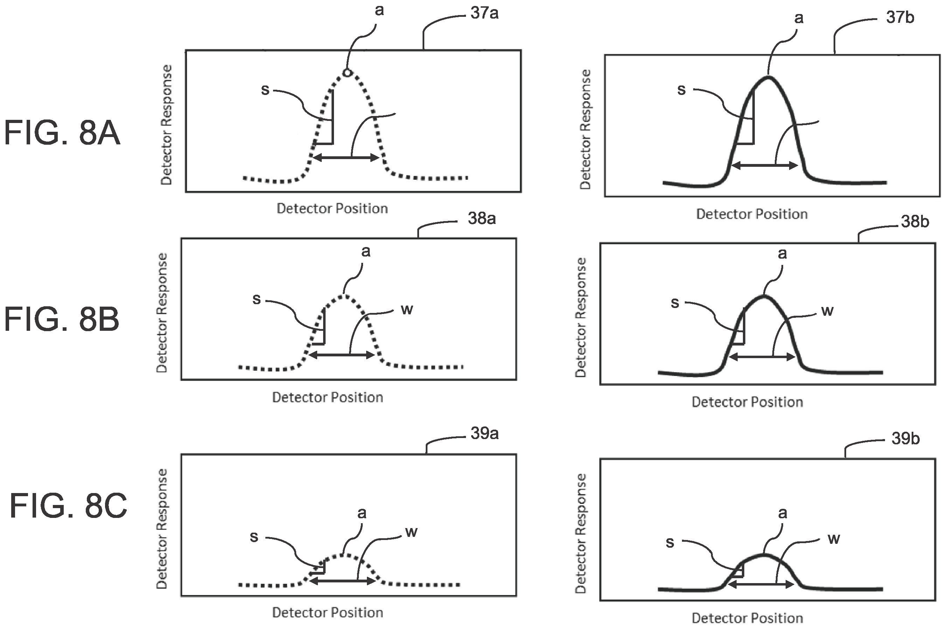

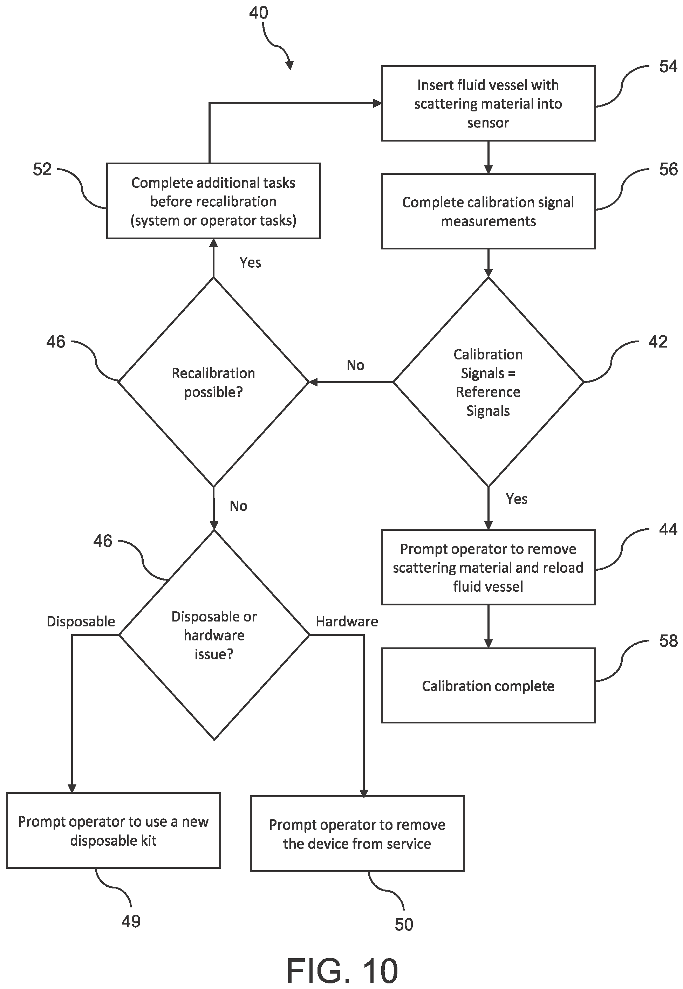

FIG. 10 sets forth the steps of a method for calibrating an optical sensing system 10. In an example, controller 18 is configured to calibrate the optical sensing system 10. To calibrate the optical sensing system 10, after receiving the collected signal from the light detector 16, the controller is configured to compare the collected signal to the reference signal (block 42). The controller 18 can compare the collected signal with the reference signal in a variety of ways. For example, controller 18 can compare various measurements of the signals to one another. For instance, when the reference signal and collected signal are measured as a function of detector position and detector response, as shown in FIGS. 8A-8C, controller 18 can compare the apex of the signals (a), the width between two data points (w), and/or the slope of the signal between two or more data points (s). It will be understood that other suitable measurements can be compared to determine if the signals are similar without departing from the scope of the disclosure.

FIGS. 8A-8C show examples of reference signals 37a, 38a, and 39a and collected signals 37b, 38b, and 39b that are, respectively, similar to one another *(i.e.,* reference signal 37a is similar to collected signal 37b, reference signal 38a is similar to collected signal 38b, and reference signal 39a is similar to collected signal 39b). If the collected signal is similar to or equal to the reference signal, the controller 18 can provide a prompt to the operator to remove the scattering material 34 and reload the fluid vessel 12 to the reusable processing device (if removed when removing the scattering material 34) (block 44). In an example, if the collected signal and the reference signal are within a predetermined threshold value of one another, such as, but not limited to, within +/-5% of one another, the reference signal and collected signal can be considered to be similar and the optical sensing system 10 can be considered calibrated. In particular, if the measurement of the collected signal (e.g., the apex of the signals (a), the width between two data points (w), or the slope of the signal between two or more data points (s)) is within +/-5% to the corresponding measurement of the reference signal, the signals can be considered similar.

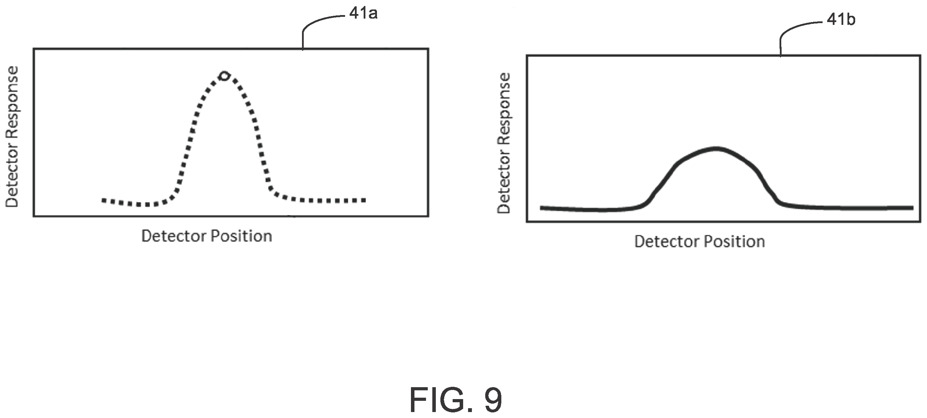

Turning to FIG. 9, the figure shows an example of a reference signal 41a and collected signal 41b that are "dissimilar" or not similar to one another. If the collected signal is dissimilar to the reference signal by a threshold amount, the controller 18 is configured to determine if recalibration is possible (FIG. 10) (block 46). The controller 18 can perform a system check to determine if recalibration is possible. For example, the controller can be configured to determine the percent error ("% error) of the collected signal to the reference signal. If the % error falls within a predetermined threshold range, recalibration can be possible, yet if the % error falls outside of the predetermined threshold range, recalibration may not be possible. In an example, if the collected signal is within +/- 5%-15%, including +/- 5% and +/- 15%, of the reference signal, then recalibration is possible. In particular, if the measurement of the collected signal (e.g., the apex of the signals (a), the width between two data points (w), or the slope of the signal between two or more data points (s)) is within +/- 5%-15%, including +/-5% and +/-15%, of the corresponding measurement of the reference signal, recalibration is possible. Alternatively, if the collected signal is greater than +/- 15% of the reference signal, recalibration is not possible. In particular, if the measurement of the collected signal (e.g., the apex of the signals (a), the width between two data points (w), or the slope of the signal between two or more data points (s)) is greater than +/-15% of the corresponding measurement of the reference signal, recalibration is not possible.

If recalibration is not possible, controller 18 can check various components of the reusable hardware device and the disposable circuit for issues preventing recalibration (block 48). The controller can be configured to use the % error of the collected signal to the reference signal to determine which components of the system (the reusable hardware device or disposable circuit) to check. In an example, large deviations between the collected signal and the reference signal, and in particular, between the apex and slope measurements, in the negative direction can signify an issue with the hardware component. In particular, if the collected signal measurement *(e.g.,* the apex (a) or slope (s)) is greater than -25% of the corresponding reference signal measurement, the controller is configured to check the reusable hardware component for issues. Such issues can include, but are not limited to, a failing light source, dirty windows in the hardware assembly, and misalignment of the optical sensor components. If an issue is detected with the reusable hardware device, controller 18 can prompt the operator to remove the device from (block 50). Alternatively, if the collected signal is within +/- 15%-25%, including +/- 25%, of the reference signal, the controller is configured to check the disposable circuit for issues. Such an issue can include, but is not limited to, clarity of the fluid passage 22. If an issue is detected with the disposable circuit, controller 18 can prompt the operator to use a new disposable fluid circuit (block 49). Controller 18 can provide other suitable prompts without departing from the scope of the disclosure. It will be understood that the predetermined threshold ranges and values described above can vary without departing from the scope of the disclosure.

If recalibration is possible, controller 18 can complete additional tasks before recalibration (block 52). For example, if the collected signal indicates a lower intensity in the collected signal compared to the reference signal (as shown in FIG. 9), controller 18 may prompt the operator to clean the surfaces of the sensor hardware *(e.g.,* the light source 14 or light detector 16) or scattering material 34. Alternatively or additionally, controller 18 may increase the baseline intensity of the light source 14 to increase the strength of the collected signal. Other actions can be taken without departing from the scope of the disclosure.

After determining that recalibration is possible and taking any additional steps before recalibrating, the fluid vessel 12 including the scattering material 34 can be re-inserted onto the reusable hardware device (if removed) (block 54), and controller 18 can complete calibration signal measurements (block 56) by directing the light source 14 to pass light through the scattering material 34 and fluid vessel 12. Controller 18 is then configured to, again, compare the reference signal to the collected signal as described herein. If the collected signal and reference signal are similar or equal to one another, calibration of the optical sensing system 10 is complete (block 58). Controller 18 can recalibrate the optical sensing system 10 as many times as necessary until the reference signal and collected signal are similar or equal to each other.

After the optical sensing system 10 has been calibrated, the operator can remove the scattering material 34 from the fluid vessel 12, replace the fluid vessel 12 onto the reusable hardware device (if removed when removing the scattering material 34), and execute the biological fluid processing procedure (e.g., under the direction of controller 18).

There are additional aspects to the devices and methods described herein including, without limitation the following aspects.

Aspect 1. A system for calibrating an optical sensor used in a blood processing procedure including a fluid vessel including a fluid passage configured to accommodate a biological fluid; an optical sensing system associated with the fluid vessel, wherein the optical sensing system includes a light source and an optical sensor; and a scattering material located on a surface of the fluid vessel.

Aspect 2. The system of Aspect 1, wherein the fluid vessel is located between the light source and the optical sensor.

Aspect 3. The system of any one of Aspects 1-2, wherein the scattering material is configured to produce a preset reference signal when exposed to a light including known properties.

Aspect 4. The system of any one of Aspects 1-3, wherein the fluid vessel includes a first surface and a second surface and the scattering material is located on the first surface and the second surface.

Aspect 5. The system of any one of Aspects 1-4, wherein the scattering material is a film.

Aspect 6. The system of Aspect 5, wherein the film is temporarily attached to the fluid vessel.

Aspect 7. The system of any one of Aspects 1-4, wherein the scattering material is a plastic sleeve.

Aspect 8. The system of Aspect 7, wherein the plastic sleeve is formed of a rigid material.

Aspect 9. The system of any one of Aspects 7-8, wherein the plastic sleeve is U-shaped.

Aspect 10. The system of any one of Aspects 1-9, wherein the fluid vessel is a cuvette configured to be placed in fluid communication with a blood processing circuit.

Aspect 11. A method of calibrating an optical sensing system including attaching a scattering material onto a surface of a fluid vessel, wherein the fluid vessel includes a fluid passage configured to accommodate a biological fluid and the scattering material is configured to produce a preset reference signal when exposed to a light including known properties; placing the fluid vessel in an optical sensing system, wherein the optical sensing system includes a light source and an optical sensor; passing a light from the light source through the fluid vessel; and comparing a collected signal from the optical sensor to the preset reference signal.

Aspect 12. The method of Aspect 11, including adjusting the light source if the collected signal is not similar to the preset reference signal.

Aspect 13. The method of any one of Aspects 11-12, including placing the fluid vessel between the light source and the optical sensor.

Aspect 14. The method of any one of Aspects 11-13, wherein the fluid vessel includes a first surface and a second surface and the scattering material is located on the first surface and the second surface.

Aspect 15. The method of any one of Aspects 11-14, wherein the scattering material is a film.

Aspect 16. The method of Aspect 15, including temporarily attaching the film to the fluid vessel.

Aspect 17. The method of any one of Aspects 11-14, wherein the scattering material is a plastic sleeve.

Aspect 18. The method of Aspect 17, wherein the plastic sleeve is formed of a rigid material.

Aspect 19. The method of any one of Aspects 17-18, wherein the plastic sleeve is U-shaped.

Aspect 20. The method of any one of Aspects 17-19, including placing the fluid vessel within the plastic sleeve.

Aspect 21. The method of any one of Aspects 11-20, wherein the fluid vessel is a cuvette configured to be placed in fluid communication with a blood processing circuit.

Aspect 22. A method of processing a blood product including loading a blood processing circuit onto a blood processing device, wherein the blood processing circuit includes a fluid vessel configured to accommodate a flow of a biological fluid; attaching a scattering material onto a surface of the fluid vessel, wherein the scattering material is configured to produce a preset reference signal when exposed to a light including known properties; placing the fluid vessel in an optical sensing system, wherein the optical sensing system includes a light source and an optical sensor; passing a light from the light source through the fluid vessel; comparing a collected signal from the optical sensor with the preset reference signal; adjusting the light source if the collected signal is not similar to the preset reference signal; and executing a blood processing procedure.

Aspect 23. The method of Aspect 22, including placing the fluid vessel between the light source and the optical sensor.

Aspect 24. The method of any one of Aspects 22-23, including removing the scattering material before executing the blood processing procedure.

Aspect 25. The method of any one of Aspects 22-24, wherein the fluid vessel includes a first surface and a second surface and the scattering material is located on the first surface and the second surface.

Aspect 26. The method of any one of Aspects 22-25, wherein the scattering material is a film.

Aspect 27. The method of any one of Aspects 22-25, wherein the scattering material is a plastic sleeve.

Aspect 28. The method of Aspect 27, wherein the plastic sleeve is formed of a rigid material.

Aspect 29. The method of any one of Aspects 27-28, wherein the plastic sleeve is U-shaped.

Aspect 30. The method of any one of Aspects 27-29, including placing the fluid vessel within the plastic sleeve.

Aspect 31. The method of any one of Aspects 22-30, wherein the fluid vessel is a cuvette.

It will be understood that the embodiments and examples described above are illustrative of some of the applications or principles of the present subject matter. Numerous modifications may be made by those skilled in the art without departing from the spirit and scope of the claimed subject matter, including those combinations of features that are individually disclosed or claimed herein. For these reasons, the scope hereof is not limited to the above description but is as set forth in the following claims, and it is understood that the claims may be directed to the features thereof, including as combinations of features that are individually disclosed or claimed herein.

## Claims

1. A system for calibrating an optical sensor used in a blood processing procedure comprising:
a fluid vessel including a fluid passage configured to accommodate a biological fluid;
an optical sensing system associated with the fluid vessel, wherein the optical sensing system includes a light source and an optical sensor; and
a scattering material located on a surface of the fluid vessel.

2. The system of Claim 1, wherein the fluid vessel is located between the light source and the optical sensor.

3. The system of any one of Claims 1-2, wherein the scattering material is configured to produce a preset reference signal when exposed to a light including known properties.

4. The system of any one of Claims 1-3, wherein the fluid vessel comprises a first surface and a second surface and the scattering material is located on the first surface and the second surface.

5. The system of any one of Claims 1-4, wherein the scattering material is a film.

6. The system of any one of Claims 1-4, wherein the scattering material is a plastic sleeve.

7. The system of any one of Claims 1-6, wherein the fluid vessel is a cuvette configured to be placed in fluid communication with a blood processing circuit.

8. A method of calibrating an optical sensing system comprising:
attaching a scattering material onto a surface of a fluid vessel, wherein the fluid vessel includes a fluid passage configured to accommodate a biological fluid and the scattering material is configured to produce a preset reference signal when exposed to a light including known properties;
placing the fluid vessel in an optical sensing system, wherein the optical sensing system includes a light source and an optical sensor;
passing a light from the light source through the fluid vessel; and
comparing a collected signal from the optical sensor to the preset reference signal.

9. The method of Claim 8, comprising adjusting the light source if the collected signal is not similar to the preset reference signal.

10. The method of any one of Claims 8-9, comprising placing the fluid vessel between the light source and the optical sensor.

11. The method of any one of Claims 8-10, wherein the fluid vessel comprises a first surface and a second surface and the scattering material is located on the first surface and the second surface.

12. The method of any one of Claims 8-11, wherein the scattering material is a film.

13. The method of any one of Claims 8-11, wherein the scattering material is a plastic sleeve.

14. The method of of Claim 13, comprising placing the fluid vessel within the plastic sleeve.

15. The method of any one of Claims 8-14, wherein the fluid vessel is a cuvette configured to be placed in fluid communication with a blood processing circuit.
